# EUROPÄISCHE PATENTSCHRIFT

(11) **EP 4 133 267 B1**
(45) Veröffentlichungstag und Bekanntmachung des Hinweises auf die Patenterteilung: **04.06.2025**
(21) Anmeldenummer: 21706547.3
(22) Anmeldetag: 17.02.2021
(51) Int. Cl.: G01N 33/00

(54) **GASMESSGERÄT MIT EINER KOMPAKTEN BAUFORM**
GAS METER WITH A COMPACT DESIGN
COMPTEUR DE GAZ À CONCEPTION COMPACTE

(30) Priorität: 08.04.2020 DE 102020109887
(43) Veröffentlichungstag der Anmeldung: 15.02.2023
(73) Patentinhaber: Opus Inspection, Inc., East Granby, CT 06026-9720 (US)
(72) Erfinder: FRANK, Holger, 79822 Titisee-Neustadt (DE)
(74) Vertreter: Bauer, Dirk
(86) Internationale Anmeldenummer: PCT/EP2021/053829
(87) Internationale Veröffentlichungsnummer: WO 2021/204448

(56) Entgegenhaltungen:
- US-A- 6 015 533
- US-A1- 2014 309 947
- US-A1- 2018 275 116

## Beschreibung

Die Erfindung betrifft ein Gasmessgerät mit wenigstens einem Messkanal, in dem wenigstens eine Sensoreinheit angeordnet ist.

Gasmessgeräte dienen beispielsweise der Abgasuntersuchung oder Brenngasuntersuchung auf bestimmte Substanzen bzw. Aerosole oder Partikel. Dabei werden üblicherweise alle Komponenten in dem Gasmessgerät über Schläuche verbunden. Die Schläuche können jedoch abknicken oder abgequetscht werden. Insbesondere bei einer komplexen Bauform eines Gasmessgeräts können die Schläuche falsch angeschlossen werden oder sich von der Verbindungsstelle lösen. Dies kann die Abgasuntersuchung erschweren.

Aus dem Schlauchmaterial können sich Weichmacher lösen und das Material wird spröde und undicht. Die Querschnitte der Schläuche an den Übergängen sind unterschiedlich, dadurch ergeben sich unterschiedliche Flussgeschwindigkeiten und Verwirbelungen. Dies kann dazu führen, dass die Analyseergebnisse verfälscht und die Abgasuntersuchungen erschwert werden.

Bei den herkömmlichen Gasmessgeräten werden typischerweise verschiedene Materialen eingesetzt. Durch eine Komponentenanordnung in dem Gasmessgerät über eine Verschlauchung wird in der Regel viel Bauraum benötigt, so dass eine platzsparende Komponentenanordnung erschwert werden kann, wodurch auch hohe Herstellungskosten entstehen können.

US 2014/309947 A1 offenbart ein Gasmessgerät, worin zwei ebene Teile einen Messkanal bilden.

Vor diesem Hintergrund ist es Aufgabe der vorliegenden Erfindung, ein Gasmessgerät mit einer vereinfachten und kompakten Bauform bereitzustellen.

Zur Lösung dieser Aufgabe sind erfindungsgemäß die Merkmale von Anspruch 1 vorgesehen. Insbesondere wird somit erfindungsgemäß zur Lösung der genannten Aufgabe bei einem Gasmessgerät der eingangs beschriebenen Art vorgeschlagen, dass das Gasmessgerät einen Grundkörper mit einem ersten Teil und einem zweiten Teil hat, wobei der erste Teil mit dem zweiten Teil an einer Schnittstelle zusammengesetzt ist und der wenigstens eine Messkanal in der Schnittstelle ausgebildet ist, wobei in dem wenigstens einen Messkanal wenigstens eine Kapillare angeordnet ist, und die wenigstens eine Kapillare als Einsetzteil ausgebildet ist.

Von Vorteil ist dabei, dass der Gasweg über einen Grundkörper und nicht über eine Verschlauchung realisierbar ist. Somit ist die Anordnung von verschiedenen Komponenten im Grundkörper auf einfache Weise ohne Verschraubung erreichbar. Dies ermöglicht die Ausbildung einer kompakten Funktionalität. Vorteilhaft ist dabei, dass ein Abknicken etwaiger Schläuche bzw. Lösen der Schläuche vom Schlauchanschluss dabei unmöglich ist, da keine Schläuche vorhanden sind. Die Erfindung macht sich zunutze, dass durch die kompakte und platzsparende Bauweise der Baumraum sehr klein gehalten werden kann, wodurch sich die Herstellungskosten reduzieren lassen können. Besonders günstig ist es, wenn die Schnittstelle flächig ausgebildet ist. Somit ist ein verhältnismäßig dichter Abschluss des wenigstens einen Messkanals erreichbar. Alternativ oder zusätzlich kann die Schnittstelle eben ausgebildet sein. Somit ist eine einfache Trennebene definierbar, die den ersten Teil von dem zweiten Teil trennt. Eine vorteilhafte Ausführungsform gemäß der Erfindung sieht vor, dass in dem Grundkörper zusätzlich wenigstens ein Kanal eines Verdünnungsstroms ausgebildet ist. Somit kann das Messgas verdünnt werden, um an die Empfindlichkeit des Sensors angepasst werden zu können.

Eine vorteilhafte Ausführungsform gemäß der Erfindung sieht vor, dass der zweite Teil des Grundkörpers als Deckel ausgebildet ist. Es ist vorgesehen, dass der erste Teil und der zweite Teil des Grundkörpers mittels einer stoffschlüssigen Verbindung zusammengesetzt sind. Die Erfindung macht sich zunutze, dass durch die kompakte Bauweise die einzelnen Komponenten stoffschlüssig in dem Gasmessgerät integriert werden können. Dadurch bleiben die Komponenten bei Erschütterungen und Schlägen an ihrer Position.

Eine vorteilhafte Ausführungsform gemäß der Erfindung sieht vor, dass die oder eine stoffschlüssige Verbindung der Schnittstelle mittels Laserschweißen gebildet ist. Alternativ kann die stoffschlüssige Verbindung mittels eines anderen Schweißverfahrens erfolgen. Dies hat den Vorteil, dass keine aufwendige Verschlauchung gesteckt oder montiert werden muss. Dadurch können Fertigungsfehler vermieden werden. Insbesondere kann der eine der beiden Teile des Grundkörpers transparent ausgebildet sein. Alternativ oder zusätzlich kann der andere Teil des Grundkörpers lichtundurchlässig ausgebildet sein. Eine vorteilhafte Ausführungsform gemäß der Erfindung sieht vor, dass der Grundkörper als Träger für wenigstens ein weiteres Verbindungselement ausgebildet ist, das über wenigstens einen Anschlussstutzen an den Messkanal angeschlossen ist.

Die Erfindung sieht vor, dass in dem wenigstens einen Messkanal wenigstens eine Kapillare angeordnet ist. Vorteilhaft ist dabei, dass die wenigstens eine Kapillare als Düse den Messstrom regulieren kann.

Die Erfindung sieht vor, dass die wenigstens eine Kapillare als Einsetzteil ausgebildet ist. Insbesondere ist vorgesehen, dass eine abdichtende Wand der wenigstens einen Kapillare in Richtung der Entformung eines Spritzgussteils ausgerichtet ist. Auf diese Weise kann ohne Schiebeteile eine Spritzgussherstellung ermöglicht werden. Alternativ oder zusätzlich kann die wenigstens eine Kapillare über den wenigstens einen Anschlussstutzen und das wenigstens eine Verbindungselement von außen wieder zurück in den wenigstens einen Messkanal verbunden werden sein. Von Vorteil ist dabei, dass auch Kreuzungspunkte im Verlauf der Messkanäle ausgebildet werden können.

Eine vorteilhafte Ausführungsform gemäß der Erfindung sieht vor, dass der wenigstens eine Messkanal wenigstens eine Pumpe aufweist, die zur Erzeugung eines Volumenstroms ausgebildet ist. Die Pumpe kann beispielsweise eine Membranpumpe sein. Vorteilhaft ist dabei, dass somit ein ausreichender Volumenstrom realisiert werden kann und Schädigungen durch etwaige flüssige Substanzen im Gas vermieden werden können.

Insbesondere ist vorgesehen, dass der Volumenstrom wenigstens 100 ml pro Minute, vorzugsweise wenigstens einen Normliter pro Minute, beträgt. Die Erfindung macht sich zunutze, dass zum einen hier Gase verarbeitet werden und zum anderen die Massenströme viel größer sind, als beispielsweise in der Mikrofluidik.

Eine vorteilhafte Ausführungsform gemäß der Erfindung sieht vor, dass in dem wenigstens einen Messkanal ein Dämpfungsvolumen von wenigstens 75 ml angeordnet ist. Vorteilhaft ist dabei, dass es somit den erzeugten Pumpstößen entgegengewirkt werden kann, wobei die Pulse abgedämpft werden können. Alternativ oder zusätzlich kann in dem wenigstens einem Messkanal ein Massenflussmesser angeordnet sein.

Eine vorteilhafte Ausführungsform gemäß der Erfindung sieht vor, dass die wenigstens eine Sensoreinheit einen Partikelabscheider (Impaktor) umfasst. Von Vorteil ist dabei, dass durch einen umgeleiteten Luftstrom oder umgelenkten Luftstrom schwere Partikel abgeschieden und deren Masse oder Häufigkeit ausgewertet werden kann.

Eine vorteilhafte Ausführungsform gemäß der Erfindung sieht vor, dass die wenigstens eine Sensoreinheit einen optochemischen Sensor umfasst. Dadurch kann die Detektion von gasförmigen, beispielsweise toxischen und/oder brennbaren, Stoffen ermöglicht werden. Alternativ oder zusätzlich kann die wenigstens eine Sensoreinheit einen optoelektronischen Sensor umfassen. Beispielsweise ist dabei auch Streulichtmessung vorgesehen. Somit kann eine präzise Partikelgrößenanalyse ermöglicht werden.

Die Erfindung wird nun anhand von Ausführungsbeispielen näher beschrieben, ist aber nicht auf diese Ausführungsbeispiele beschränkt. Weitere Ausführungsbeispiele ergeben sich durch Kombination der Merkmale einzelner oder mehrerer Schutzansprüche untereinander und/oder mit einzelnen oder mehreren Merkmalen der Ausführungsbeispiele.

Es zeigt:
- Fig. 1: ein Gasmessgerät mit einem Grundkörper aus zwei Teilen in einer Schrägansicht von der Seite,
- Fig. 2: ein Gasmessgerät mit einem Grundkörper aus zwei Teilen gemäß Fig. 1 in einem Querschnitt in X- und Z-Achse, wobei ein Messkanal erkennbar ist,
- Fig. 3: ein Gasmessgerät mit einem Grundkörper aus zwei Teilen gemäß Fig. 1 in einem Querschnitt in Y- und Z-Achse, wobei das Zusammensetzen des ersten Teils mit dem zweiten Teil an einer Schnittstelle erkennbar ist, in welcher ein Messkanal ausgebildet ist,
- Fig. 4: ein Gasmessgerät mit einem Grundkörper aus zwei Teilen gemäß Fig. 1 in einem Querschnitt in X- und Y-Achse, wobei eine in dem Messkanal angeordnete Kapillare erkennbar ist.

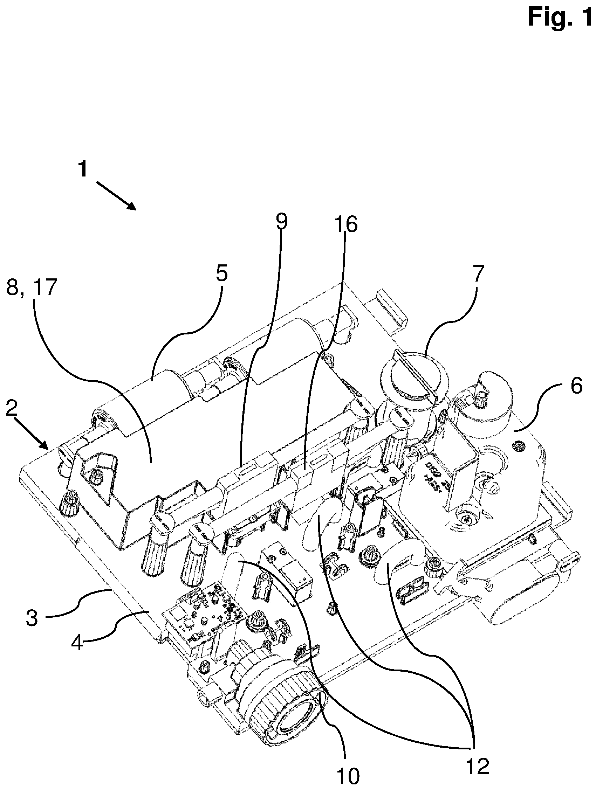

In den Figuren 1 bis 4 ist ein Gasmessgerät 1 mit einem Grundkörper 2 in verschiedenen Ausführungen dargestellt.

Fig. 1 zeigt ein Gasmessgerät 1 mit einem Grundkörper 2 in einer Schrägansicht von der Seite. Aus Fig. 1 ist erkennbar, dass das Gasmessgerät 1 einen Grundkörper 2 mit einem ersten Teil 3 und einem zweiten Teil 4 umfasst. Der zweite Teil 4 des Grundkörpers 2 ist als Deckel ausgebildet.

Der erste Teil 3 und der zweite Teil 4 des Grundkörpers 2 werden durch atomare oder molekulare Kräfte zusammengehalten, so dass eine stoffschlüssige Verbindung entsteht, wobei sich die Verbindungspartner nur durch Zerstörung der Verbindungsmittel trennen lassen.

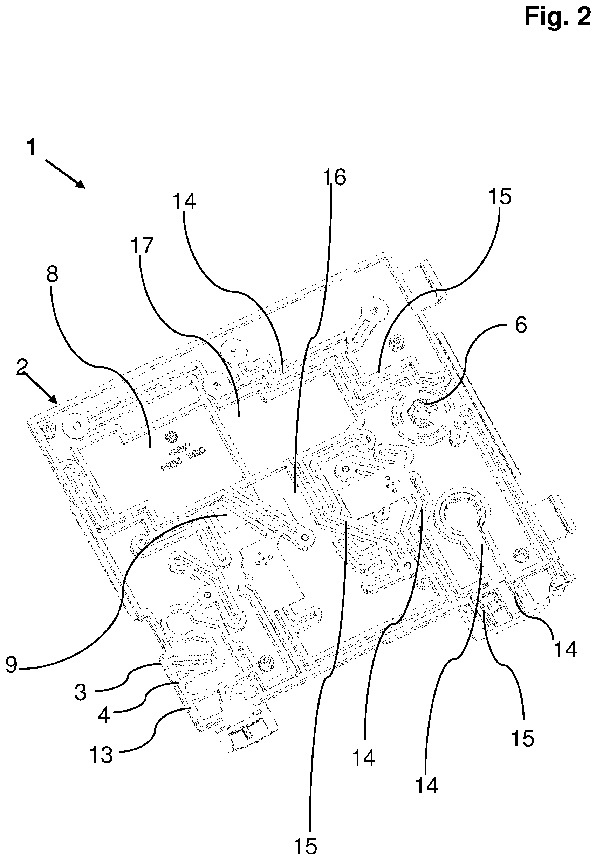

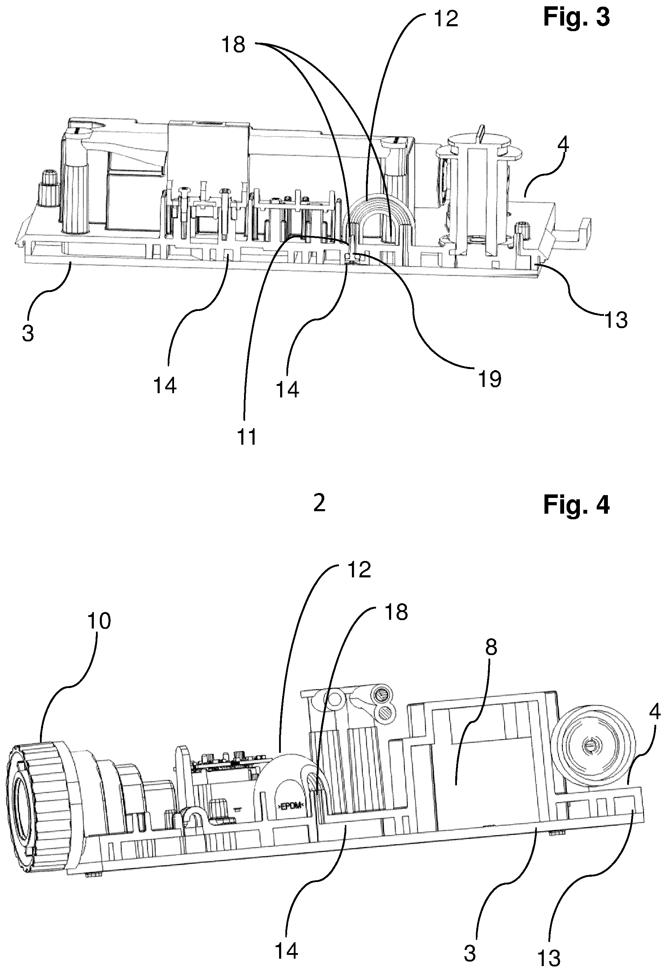

Die Fig. 1 bis 4 zeigen, dass einzelne Komponenten des Gamessgeräts 1 in dem Grundkörper 2 eingebettet und umschlossen sind. Bei den Ausführungsbeispielen nach Fig. 1 und Fig. 2 ist ersichtlich, dass die Komponente ein Filter 5, eine Pumpe 6, eine weitere Pumpe 7, ein Dämpfungsvolumen 8, ein Massenflussmesser 9 und ein Partikelabscheider (Impaktor) 10 sein kann. Weitere Beispiele für die Komponente des Gasmessgeräts 1 können eine Kapillare 11 und ein Verbindungselement 12 darstellen, wie in den Fig. 3 und 4 gezeigt.

Aus Fig. 2 bis 4 ist weiterhin ersichtlich, dass der erste Teil 3 mit dem zweiten Teil 4 an einer vorzugsweise flächigen und/oder ebenen Schnittstelle 13 zusammengesetzt ist, in welcher ein Messkanal 14 ausgebildet ist. Durch den ausgebildeten Messkanal 14 kann das Messgas über eine Pumpe 6 und einen Massenflussmesser 9 zum Dämpfungsvolumen 8 geleitet werden, wie aus Fig. 2 ersichtlich. Die Pumpe 6 ist zur Erzeugung eines Volumenstroms ausgebildet. Das in dem Grundkörper 2 angeordnete Dämpfungsvolumen 8 kann den erzeugten Pumpstößen entgegenwirken, so dass die Pulse abgedämpft werden.

Um das Messgas zu verdünnen und somit an die Empfindlichkeit des Sensors anzupassen, ist in dem Grundkörper zusätzlich zu dem wenigstens einem Messkanal 14 wenigstens ein Kanal eines Verdünnungsstroms 15 ausgebildet, wie die Fig. 2 zeigt. Entsprechend befinden sich in dem Grundkörper 2 eine weitere Pumpe 7, ein weiterer Massenflussmesser 16 und ein weiteres Dämpfungsvolumen 17 für das Verdünnungsgas.

Die Fig. 1, 3 und 4 zeigen, dass in dem Grundkörper wenigstens ein Verbindungselement 12 ausgebildet ist, das über wenigstens einen Anschlussstutzen 18 an den Messkanal 14 angeschlossen ist. Bei dem Ausführungsbeispiel nach Fig. 1 ist ersichtlich, dass in dem Grundkörper 2 drei Verbindungselemente 12 mit jeweils zwei Anschlussstutzen 18 ausgebildet sind. Bei weiteren Ausführungsbeispielen ist eine andere Anzahl von Verbindungselementen 12, beispielsweise mehr als drei oder weniger als drei, und eine andere Anzahl von Anschlussstutzen 18, beispielsweise mehr als zwei oder weniger als zwei, vorhanden. Somit kann der Messkanal 14 bei besonderen Platzverhältnissen über vorhandene Anschlussstutzen wahlweise an die entsprechenden Verbindungselemente angeschlossen werden.

Aus Fig. 3 ist weiterhin ersichtlich, dass in dem Messkanal 14 eine Kapillare 11 angeordnet ist. Die Kapillare 11 kann als

Düse den Messstrom regulieren. Die Kapillare 11 und der Messkanal 14 sind durch eine Dichtung 19 gasdicht gegeneinander abgedichtet. Damit auch Kreuzungspunkte im Verlauf des Messkanals ausgebildet werden können, ist die Kapillare 11 über den Anschlussstutzen 18 und das Verbindungselement 12 von außen wieder zurück in den Messkanal 14 verbunden, wie in der Fig. 3 dargestellt.

Die Fig. 1 zeigt weiterhin, dass eine in dem Messkanal 14 angeordnente, hier nicht dargestellte Sensoreinheit einen Partikelabscheider (Impaktor) 10 zur Ermittlung von Partikeln im Messgas umfasst.

Erfindungsgemäß wird somit vorgeschlagen, ein Gasmessgerät 1 mit wenigstens einem Messkanal 14, in dem wenigstens eine Sensoreinheit angeordnet ist, bereitzustellen, wobei das Gasmessgerät 1 einen Grundkörper 2 mit einem ersten Teil 3 und einem zweiten Teil 4 hat, wobei der erste Teil 3 mit dem zweiten Teil 4 an einer vorzugsweise flächigen und/oder ebenen Schnittstelle 13 zusammengesetzt ist und der wenigstens eine Messkanal 14 in der Schnittstelle 13 ausgebildet ist, wobei in dem wenigstens einen Messkanal 14 wenigstens eine Kapillare 11 angeordnet ist und die wenigstens eine Kapillare 11 als Einsetzteil ausgebildet ist.

### Bezugszeichenliste

- 1: Gasmessgerät
- 2: Grundkörper
- 3: erster Teil des Grundkörpers 2
- 4: zweiter Teil des Grundkörpers 2
- 5: Filter
- 6: Pumpe
- 7: Weitere Pumpe
- 8: Dämpfungsvolumen
- 9: Massenflussmesser
- 10: Partikelabscheider (Impaktor)
- 11: Kapillare
- 12: Verbindungselement
- 13: Schnittstelle
- 14: Messkanal
- 15: Kanal eines Verdünnungsstroms
- 16: weiterer Massenflussmesser
- 17: weiteres Dämpmfungsvolumen
- 18: Anschlussstutzen
- 19: Dichtung

## Patentansprüche

1. Gasmessgerät (1) mit wenigstens einem Messkanal (14), in dem wenigstens eine Sensoreinheit angeordnet ist, wobei das Gasmessgerät (1) einen Grundkörper (2) mit einem ersten Teil (3) und einem zweiten Teil (4) hat, wobei der erste Teil (3) mit dem zweiten Teil (4) an einer vorzugsweise flächigen und/oder ebenen Schnittstelle (13) zusammengesetzt ist und der wenigstens eine Messkanal (14) in der Schnittstelle (13) ausgebildet ist, **dadurch gekennzeichnet, dass** in dem wenigstens einen Messkanal (14) wenigstens eine Kapillare (11) angeordnet ist und die wenigstens eine Kapillare (11) als Einsetzteil ausgebildet ist.

2. Gasmessgerät (1) nach Anspruch 1, **dadurch gekennzeichnet, dass** in dem Grundkörper (2) zusätzlich wenigstens ein Kanal eines Verdünnungsstroms (15) ausgebildet ist.

3. Gasmessgerät (1) nach einem der vorangehenden Ansprüche, **dadurch gekennzeichnet, dass** der zweite Teil (4) des Grundkörpers (2) als Deckel ausgebildet ist, wobei der erste Teil (3) und der zweite Teil (4) des Grundkörpers (2) mittels einer stoffschlüssigen Verbindung zusammengesetzt sind.

4. Gasmessgerät (1) nach dem vorangehenden Anspruch, **dadurch gekennzeichnet, dass** die stoffschlüssige Verbindung mittels Laserschweißen gebildet ist, insbesondere wobei der eine der beiden Teile des Grundkörpers (2) transparent und/oder der andere Teil lichtundurchlässig ausgebildet ist.

5. Gasmessgerät (1) nach einem der vorangehenden Ansprüche, **dadurch gekennzeichnet, dass** der Grundkörper (2) als Träger für wenigstens ein weiteres Verbindungselement (12) ausgebildet ist, das über wenigstens einen Anschlussstutzen (18) an den Messkanal (14) angeschlossen ist.

6. Gasmessgerät (1) nach einem der vorangehenden Ansprüche, **dadurch gekennzeichnet, dass** eine abdichtende Wand der wenigstens einen Kapillare (11) in Richtung der Entformung eines Spritzgussteils ausgerichtet ist und/oder die wenigstens eine Kapillare (11) über den wenigstens einen Anschlussstutzen (18) und das wenigstens eine Verbindungselement (12) von außen wieder zurück in den wenigstens einen Messkanal (14) verbunden ist.

7. Gasmessgerät (1) nach einem der vorangehenden Ansprüche, **dadurch gekennzeichnet, dass** der wenigstens eine Messkanal (14) wenigstens eine Pumpe (6), vorzugsweise eine Membranpumpe, aufweist, die zur Erzeugung eines Volumenstroms ausgebildet ist, insbesondere wobei der Volumenstrom wenigstens 100 ml pro Minute, vorzugsweise wenigstens einen Normliter pro Minute, beträgt.

8. Gasmessgerät (1) nach einem der vorangehenden Ansprüche, **dadurch gekennzeichnet, dass** in dem wenigstens einen Messkanal (14) ein Dämpfungsvolumen (8) von wenigstens 75 ml und/oder ein Massenflussmesser (9) angeordnet ist.

9. Gasmessgerät (1) nach einem der vorangehenden Ansprüche, **dadurch gekennzeichnet, dass** die wenigstens eine Sensoreinheit einen Partikelabscheider (10) umfasst.

10. Gasmessgerät (1) nach einem der vorangehenden Ansprüche, **dadurch gekennzeichnet, dass** die wenigstens eine Sensoreinheit einen optochemischen und/oder optoelektronischen Sensor umfasst.

## Claims

1. A gas measuring apparatus (1), comprising:
at least one measuring channel (14), including
at least one sensor unit,
a core body (2) including a first part (3) and a second part (4),
wherein the first part (3) is assembled at the second part (4) at a flat or planar interface (13) and the at least one measuring channel (14) is configured in the flat or planar interface (13),
**characterized in that** at least one capillary (11) is arranged in the at least one measuring channel (14) and the at least one capillary (11) is configured as an insert.

2. The gas measuring apparatus (1) according to claim 1, **characterized in that** the core body (2) includes at least one dilution flow channel (15).

3. The gas measuring apparatus (1) according to one of the preceding claims,
**characterized in that** the second part (4) of the core body (2) is configured as a cover,
wherein the first part (3) and the second part (4) of the core body (2) are bonded together by a bonded connection.

4. The gas measuring apparatus (1) according to the preceding claim,
**characterized in that** the bonded connection is formed by laser welding, and in particular,
wherein one or both parts of the core body (2) are configured transparent, and/or the other part is configured non-light permeable.

5. The gas measuring apparatus (1) according to one of the preceding claims, **characterized in that** the core body (2) supports at least one additional connection element (12) that is connected through at least one connection spout (18) to the measuring channel (14).

6. The gas measuring apparatus (1) according to one of the preceding claims,
**characterized in that** a sealing wall of the at least one capillary (11) is oriented in a mold extraction direction of an injection molded component, and/or
the at least one capillary (11) is connected through the at least one connection spout (18) and the at least one connection element (12) from an outside back into the at least one measuring channel (14).

7. The gas measuring apparatus (1) according to one of the preceding claims, **characterized in that** the at least one measuring channel (14) includes a pump (6), advantageously a membrane pump configured to generate a volume flow,
in particular, wherein the volume flow is at least 100 ml per minute, advantageously at least one standard liter per minute.

8. The gas measuring apparatus (1) according to one of the preceding claims, **characterized in that** the at least one measuring channel (14) includes a damping volume (8) of at least 75 ml and/or a mass flow measuring device (9).

9. The gas measuring apparatus (1) according to one of the preceding claims, **characterized in that** the at least one sensor unit includes a particle precipitator (10).

10. The gas measuring apparatus (1) according to one of the preceding claims, **characterized in that** the at least one sensor unit includes an opto-chemical and/or an opto-electronic sensor.

## Revendications

1. Appareil de mesure du gaz (1) avec au moins un conduit de mesure (14), dans lequel est disposé au moins une unité de détection, sachant que l'appareil de mesure du gaz (1) possède un corps de base (2) avec une première partie (3) et une deuxième partie (4), sachant que la première partie (3) est assemblée à la deuxième partie (4) sur une interface (13) de préférence en surface et/ou plane et au moins un conduit de mesure (14) est constitué dans l'interface (13), **caractérisé en ce qu'**au moins un capillaire (11) est disposé dans au moins un conduit de mesure (14) et au moins un capillaire (11) est constitué sous la forme d'une pièce d'insertion.

2. Appareil de mesure du gaz (1) selon la revendication 1, **caractérisé en ce qu'**au moins un conduit d'un écoulement de dilution (15) est en plus constitué dans le corps de base (2).

3. Appareil de mesure du gaz (1) selon l'une quelconque des revendications précédentes, **caractérisé en ce que** la deuxième partie (4) du corps de base (2) est constituée sous la forme d'un couvercle, sachant que la première partie (3) et la deuxième partie (4) du corps de base (2) sont assemblés au moyen d'une liaison par conformité de matière.

4. Appareil de mesure du gaz (1) selon l'une quelconque des revendications précédentes, **caractérisé en ce que** la liaison par conformité de matière est formée au moyen d'une soudure au laser, en particulier, sachant qu'une des deux parties du corps de base (2) est constituée transparente et/ou l'autre partie est constituée opaque.

5. Appareil de mesure du gaz (1) selon l'une quelconque des revendications précédentes, **caractérisé en ce que** le corps de base (2) est constitué sous la forme d'un support pour au moins un autre élément de liaison (12), qui est raccordé au conduit de mesure (14) par le biais d'au moins une tubulure de raccord (18).

6. Appareil de mesure du gaz (1) selon l'une quelconque des revendications précédentes, **caractérisé en ce qu'**une paroi étanchéifiante d'au moins un capillaire (11) est orientée en direction de démoulage d'une pièce moulée par injection et/ou au moins un capillaire (11) est relié par au moins une tubulure de raccord (18) et par au moins un élément de liaison (12) de l'extérieur à nouveau en retour dans au moins un conduit de mesure (14).

7. Appareil de mesure du gaz (1) selon l'une quelconque des revendications précédentes, **caractérisé en ce qu'**au moins un conduit de mesure (14) comporte au moins une pompe (6), de préférence une pompe à membrane, qui est constituée pour produire un débit, en particulier, sachant que le débit est au moins de 100 ml par minute, de préférence d'au moins un litre normalisé par minute.

8. Appareil de mesure du gaz (1) selon l'une quelconque des revendications précédentes, **caractérisé en ce qu'**un volume d'amortissement (8) d'au moins 75 ml et/ou un débitmètre de masse (9) est disposé dans au moins un conduit de mesure (14).

9. Appareil de mesure du gaz (1) selon l'une quelconque des revendications précédentes, **caractérisé en ce qu'**au moins une unité de détection comprend un séparateur de particules (10).

10. Appareil de mesure du gaz (1) selon l'une quelconque des revendications précédentes, **caractérisé en ce qu'**au moins une unité de détection comprend un capteur optochimique et/ou optoélectronique.
